# EUROPEAN PATENT APPLICATION

(11) **EP 4 461 330 A1**
(43) Date of publication of application: **13.11.2024**
(21) Application number: 22919042.6
(22) Date of filing: 14.12.2022
(51) Int. Cl.: A61M 5/158, A61M 5/142, A61M 25/06, A61M 5/145

(54) **NEEDLE ASSEMBLY AND LIQUID MEDICINE INJECTION APPARATUS COMPRISING SAME**

(30) Priority: 04.01.2022 KR 20220001049; 04.02.2022 KR 20220014997; 27.09.2022 KR 20220122887
(71) Applicant: Eoflow Co., Ltd., Bundang-gu, Seongnam-si Gyeonggi-do 13605 (KR)
(72) Inventor: YUN, Kwang Sik, Hwaseong-si Gyeonggi-do 18466 (KR); LEE, Do Kyung, Hwaseong-si Gyeonggi-do 18466 (KR); JEONG, Jun Hyuk, Yongin-si Gyeonggi-do 16918 (KR)
(74) Representative: Meissner Bolte Partnerschaft mbB
(86) International application number: PCT/KR2022/020294
(87) International publication number: WO 2023/132505

(57) **Abstract**

A needle assembly includes a needle, a cannula into which the needle is inserted, a driving member generating driving force to move the needle and the cannula, and a holder on which the driving member is supported, in which the holder rotates to move the needle and the cannula in a direction, and the holder further rotates to move the needle in another direction.

## Description

### Technical Field

The present disclosure relates to a needle assembly and a liquid medicine injection apparatus comprising same.

### Background Art

Generally, a liquid medicine injection apparatus such as an insulin injection device is used to inject medical liquid into a body of a patient. Such a liquid medicine injection apparatus is used by medical professionals like doctors or nurses, but in most cases, by ordinary persons like patients or carers for the patients.

For diabetic patients, especially, pediatric diabetic patients, medical liquid like insulin needs to be injected into a human body at set intervals. A liquid medicine injection apparatus in the form of a patch used by being attached to a human body for a specific period has been developed, and such a liquid medicine injection apparatus may be used by being attached to the human body such as an abdomen, waist, etc., of a patient, in the form of a patch for the specific period.

To increase the effect of medical liquid injection, the liquid medicine injection apparatus needs to be controlled to precisely inject the medical liquid into the body of the patient, and it is important to precisely inject a small amount of medical liquid through a small-size liquid medicine injection apparatus.

The liquid medicine injection apparatus, when attached to the human body, needs to be comfortable to wear, convenient to use, durable, and driven with low power. In particular, the liquid medicine injection apparatus is used by being directly attached to the skin of the patient, such that it is important for a user to insert a needle, a cannula, etc., into the skin of the patient conveniently.

### Detailed Description of the Invention

### Technical Problem

The present disclosure provides a needle assembly driven safely and simply.

The present disclosure provides a liquid medicine injection apparatus including a needle assembly driven safely and simply.

### Technical Solution

A needle assembly according to embodiments of the present disclosure includes a needle, a cannula into which the needle is inserted, a driving member generating driving force to move the needle and the cannula, and a holder on which the driving member is supported, in which the holder rotates to move the needle and the cannula in a direction, and the holder further rotates to move the needle in another direction.

### Effects of the Invention

In a needle assembly according to example embodiments, an operation of the needle assembly may be efficiently performed by a user's single operation of pressing a button, thereby minimizing the probability of failure during the operation of the needle assembly.

Moreover, in the needle assembly according to example embodiments, as a hole of a guide member is inclined with respect to the user's skin, the needle and the cannula may be inclinedly inserted with respect to the user's skin by the guide member. In addition, the insertion speed of the needle and the cannula into the user's skin may be higher than the withdrawal speed of the needle from the user's skin. Thus, when the needle and the cannula are inserted into the user's skin, damage to the user's skin may be minimized when the needle is withdrawn from the user's skin.

The operation of the needle assembly may also be efficiently performed by a user's single operation of pressing the sleeve, thereby minimizing the probability of failure in the operation of the needle assembly.

Accordingly, the liquid medicine injection apparatus including the needle assembly may safely and simply inject a required amount of medical liquid into a user's body.

### Brief Description of Drawings

FIG. 1 is a perspective view showing a liquid medicine injection apparatus according to example embodiments.
FIG. 2 is a perspective view showing an internal arrangement of a liquid medicine injection apparatus of FIG. 1.
FIG. 3 is a plan view showing a liquid medicine injection apparatus of FIG. 2.
FIG. 4 is a perspective view showing a needle assembly of FIG. 2.
FIGS. 5A to 7C are side views and cross-sectional views showing a driving operation of a needle assembly.
FIG. 8 is a perspective view showing a liquid medicine injection apparatus according to example embodiments.
FIG. 9 is a perspective view showing an internal arrangement of a liquid medicine injection apparatus of FIG. 8.
FIG. 10 is a plan view showing a liquid medicine injection apparatus of FIG. 9.
FIGS. 11 and 12 are side views showing a needle assembly of FIG. 9.
FIGS. 13A to 13C are perspective views showing a driving operation of a needle assembly.
FIGS. 14A to 14C are bottom views showing a driving operation of a needle assembly.

### Best Mode for Invention

A needle assembly according to embodiments of the present disclosure to achieve the foregoing object includes a needle, a cannula into which the needle is inserted, a driving member generating driving force to move the needle and the cannula, and a holder on which the driving member is supported, in which the holder rotates to move the needle and the cannula in a direction, and the holder further rotates to move the needle in another direction.

The needle assembly may further include a first sliding member to which the needle is fixed and a second sliding member to which the cannula is fixed and which is disposed adjacent to the first sliding member, in which the holder is connected to the first sliding member.

The needle assembly may further include a catching member disposed on a movement path of the first sliding member and the second sliding member, in which the second sliding member is caught and fixed to the catching member.

The holder may include, on a bottom surface thereof, a holder projection protruding downwardly, and the first sliding member may include, on a surface thereof, a curved first slot hole into which the holder projection is inserted.

The needle assembly may further include a sliding rail providing the movement path for the first sliding member and the second sliding member, in which the sliding rail includes, on a surface thereof, a curved second slot hole into which the holder projection is inserted.

The holder projection may move circularly along the first slot hole and the second slot hole.

A liquid medicine injection apparatus according to embodiments of the present disclosure to achieve the foregoing another object includes a base body, a reservoir unit mounted on the base body and storing medical liquid, a needle assembly mounted on the base body and injecting medical liquid into a body of a user, and a driving module connected to the reservoir unit to transfer the medical liquid of the reservoir unit to the needle assembly, in which the needle assembly includes a needle, a cannula into which the needle is inserted, a driving member generating driving force to move the needle and the cannula, and a holder on which the driving member is supported, in which the holder rotates to move the needle and the cannula in a direction, and the holder further rotates to move the needle in another direction.

A needle assembly according to other embodiments of the present disclosure to achieve the foregoing object includes a needle, a cannula into which the needle is inserted, a driving member generating driving force to move the needle and the cannula, a holder on which the driving member is supported, and a connector disposed between the holder and the needle, in which the connector moves the needle and the cannula in a direction when the holder rotates, and the connector moves the needle in another direction when the holder further rotates.

The needle assembly may further include a first sliding member to which the needle is fixed and a second sliding member to which the cannula is fixed and which is disposed adjacent to the first sliding member, in which the holder and the first sliding member may be connected to each other by the connector.

A movement speed of the first sliding member and the second sliding member in the direction may be different from the movement speed of the first sliding member in the other direction.

The needle assembly may further include a catching member disposed on a movement path of the first sliding member and the second sliding member, in which the second sliding member is caught and fixed to the catching member.

The connector may include a first slot hole into which a sliding projection of the first sliding member is inserted, and a second slot hole into which a holder projection of the holder is inserted.

The sliding projection may move linearly along the first slot hole, and the holder projection may move linearly along the second slot hole.

The driving member may be spaced apart from the movement path of the first sliding member and the second sliding member.

A liquid medicine injection apparatus according to other embodiments of the present disclosure to achieve the foregoing another object includes a base body, a reservoir unit mounted on the base body and storing medical liquid, a needle assembly mounted on the base body and injecting medical liquid into a body of a user, and a driving module connected to the reservoir unit to transfer the medical liquid of the reservoir unit to the needle assembly, in which the needle assembly includes a needle, a cannula into which the needle is inserted, a driving member generating driving force to move the needle and the cannula, a holder on which the driving member is supported, and a connector disposed between the holder and the needle, in which the connector moves the needle and the cannula in a direction when the holder rotates, and the connector moves the needle in another direction when the holder further rotates.

### Mode for Invention

The present disclosure may have various modifications thereto and various embodiments, and thus particular embodiments will be illustrated in the drawings and described in detail in a detailed description. Effects and features of the present disclosure, and methods for achieving them will become clear with reference to the embodiments described later in detail together with the drawings. However, the present disclosure is not limited to the embodiments disclosed below and may be implemented in various forms.

Hereinafter, embodiments of the present disclosure will be described in detail with reference to the accompanying drawings, and in description with reference to the drawings, the same or corresponding components are given the same reference numerals, and redundant description thereto will be omitted.

In the following embodiments, singular forms include plural forms unless apparently indicated otherwise contextually.

In the following embodiments, the terms "include", "have", or the like, are intended to mean that there are features, or components, described herein, but do not preclude the possibility of adding one or more other features or components.

If a certain embodiment may be implemented otherwise, a particular process order may be performed differently from the order described. For example, two processes described in succession may be performed substantially simultaneously, or may be performed in an order reverse to the order described.

In the drawings, the size of components may be exaggerated or reduced for convenience of description. For example, the size and thickness of each component shown in the drawings are shown for convenience of description, and thus the present disclosure is not necessarily limited to the illustration.

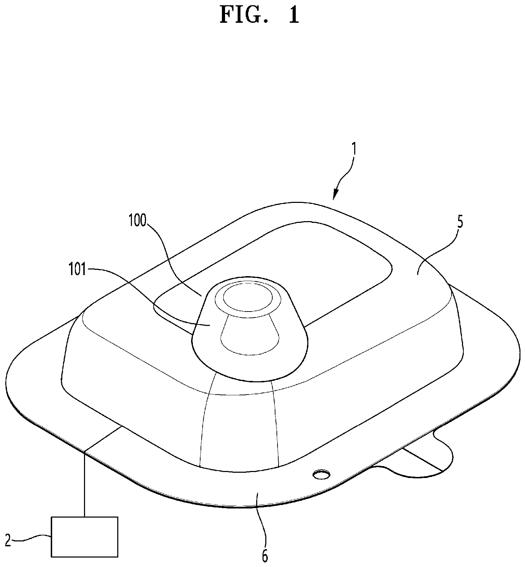

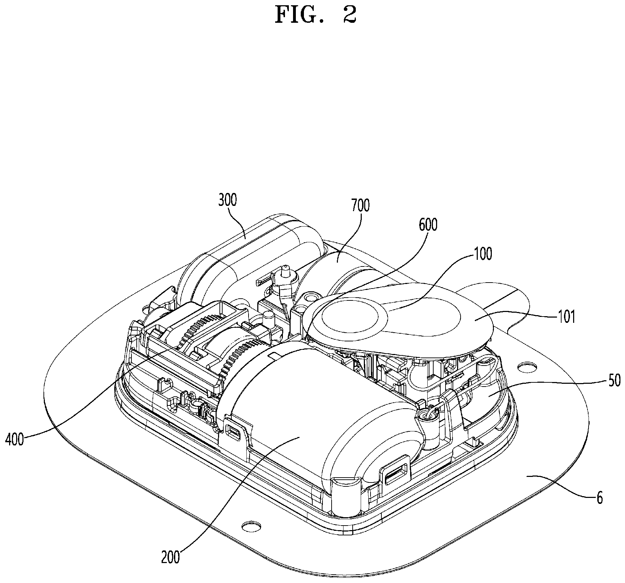

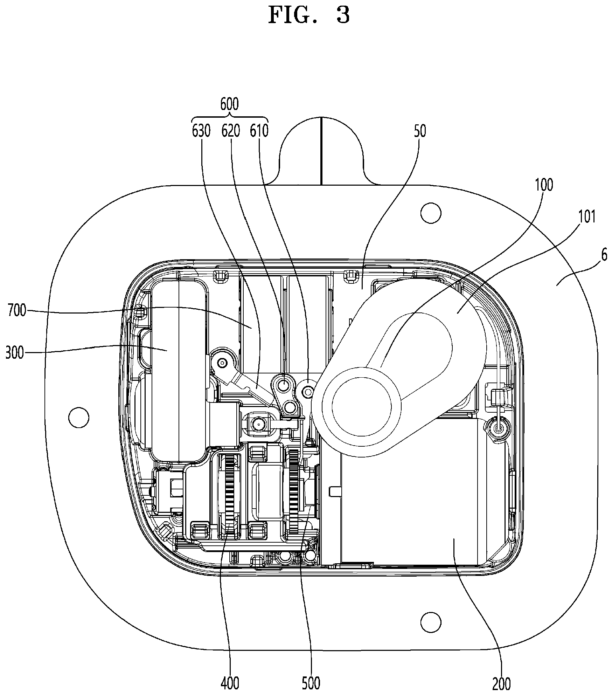

FIG. 1 is a perspective view showing a liquid medicine injection apparatus according to example embodiments, FIG. 2 is a perspective view showing an internal arrangement of the liquid medicine injection apparatus of FIG. 1, and FIG. 3 is a plan view showing the liquid medicine injection apparatus of FIG. 2.

Referring to FIGS. 1 to 3, a liquid medicine injection apparatus 1 may be attached to a skin, etc., of a user and inject medical liquid stored therein into the user in a set required amount. The liquid medicine injection apparatus 1 may be used for various purposes according to a type of the medical liquid to be injected. The liquid medicine injection apparatus 1 may be mounted on not only humans but also animals to inject the medical liquid.

The liquid medicine injection apparatus 1 may be used for various purposes according to a type of the medical liquid to be injected. For example, the medical liquid may include insulin-based medical liquid for diabetic patients, and other various types of medical liquid such as medical liquid for pancreas, medical liquid for heart, etc.

The liquid medicine injection apparatus 1 may be connected to a remote device 2 wiredly or wirelessly connected thereto. The user may control the liquid medicine injection apparatus 1 and monitor a use state of the liquid medicine injection apparatus 1, by manipulating the remote device 2. For example, the amount of the medical liquid injected from the liquid medicine injection apparatus 1, the number of injections of the medical liquid, the amount of the medical liquid stored in a reservoir unit 200, bio information of the user, etc., may be monitored, and based on the same, the user may drive the liquid medicine injection apparatus 1.

In an embodiment, the remote device 2 may refer to a communication terminal that may use an application in a wired/wireless communication environment. Herein, the remote device 2 may be a portable terminal of the user. More specifically, the remote device 2 may include, but not limited to, a computer (e.g., a desktop, a laptop, a tablet, etc.), a media computing platform (e.g., a cable, a satellite set-top box, a digital video recorder), a handheld computing device (e.g., a personal digital assistant (PDA), an e-mail client, etc.), any type of a cellular phone, any type of a wearable device that may be used by being attached or mounted on a body of the user, or any other type of computing or communication platform.

The liquid medicine injection apparatus 1 and the remote device 2 may communicate with each other through a communication network. The communication network may refer to a communication network that provides a connection path such that the remote device 2 may transmit and receive data after connecting to a service server (not shown). The communication network may include wired networks such as local area networks (LANs), wide area networks (WANs), metropolitan area networks (MANs), integrated service digital networks (ISDNs), etc., or wireless networks such as wireless LANs, code division multiple access (CDMA), Bluetooth, satellite communication, etc., but the scope of the present disclosure is not limited thereto.

While it is shown in FIG. 1 that the remote device 2 is a single device, the present disclosure is not necessarily limited thereto and may include a plurality of devices capable of communicating with the liquid medicine injection apparatus 1.

The liquid medicine injection apparatus 1 may include a housing 5 covering an outer side thereof and an attachment portion 6 positioned adjacent to the user's skin. The liquid medicine injection apparatus 1 may include a plurality of internal components arranged in an inner space between the housing 5 and the attachment portion 6. A separate bonding means may be further provided between the attachment portion 6 and the user's skin, and the liquid medicine injection apparatus 1 may be fixed to the skin by the bonding means.

The liquid medicine injection apparatus 1 may include a base body 50, a needle assembly 100, a reservoir unit 200, a driving module 300, a driving unit 400, a clutch unit 500, a trigger member 600, and a battery 700.

The base body 50 may form a basic frame of the housing 5, may be mounted in the inner space of the housing 5, and may fix the internal components of the liquid medicine injection apparatus 1 at preset positions. In an embodiment, the base body 50 may include a first body that covers a top side of the internal components and a second body that covers a bottom side of the internal components. In another embodiment, the base body 50 may be formed as one integral frame.

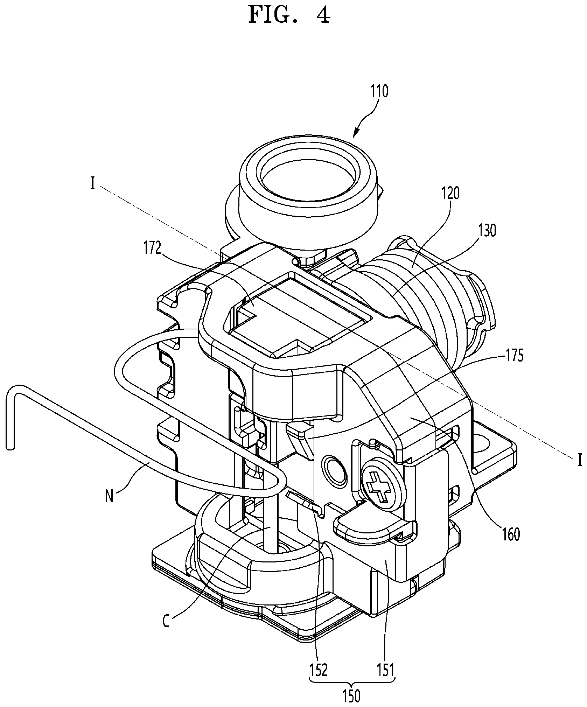

FIG. 4 is a perspective view showing the needle assembly of FIG. 2.

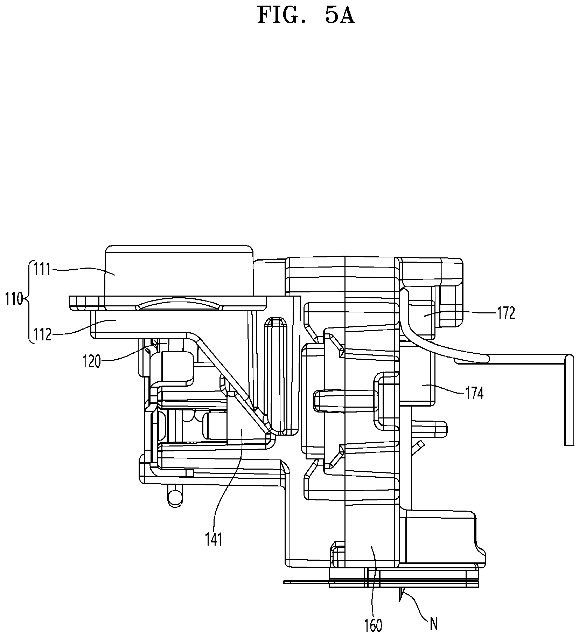

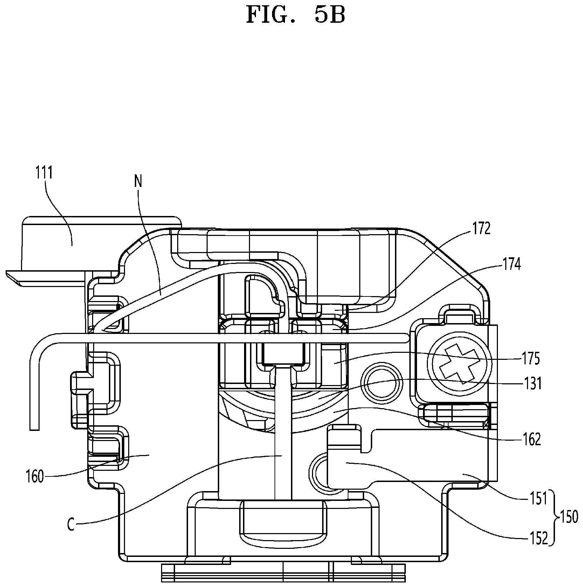

Referring to FIG. 4, the needle assembly 100 may include a needle N, a cannula C, a driving member 120, and a holder 130. Furthermore, the needle assembly 100 may further include a button 101, an elevating member 110, a fixing member 140, a catching member 150, a sliding rail 160, a first sliding member 172, a second sliding member 174, a guide member 180, and a tube T (see FIGS. 2 and 5A to 5C). Meanwhile, for convenience of understanding the structure of the needle assembly, the button 101 is not shown in FIG. 4.

The button 101 may form the exterior of the needle assembly 100.

The elevating member 110 may be disposed on a bottom surface of the button 101 and may be elevated. The elevating member 110 may include an elevating body 111 and an elevating projection 112.

The elevating body 111 may be elevated by user manipulation. In example embodiments, a user may elevate the elevating body 111 by manipulating the button 101 exposed to outside.

The elevating projection 112 may be disposed adjacent to the fixing member 140 under the elevating body 111. The elevating projection 112 may be elevated and lowered together as the elevating body 111 is elevated and lowered, such that the fixing member 140 may fix and rotate the holder 130. Details of how the fixing member 140 fixes and rotates the holder 130 will be described in detail later.

In an embodiment, one surface of the elevating projection 112 adjacent to the fixing member 140 may be inclined with respect to a elevating direction. In an embodiment, the width of a portion of the elevating projection 112 adjacent to the fixing member 140 in a horizontal direction substantially perpendicular to the elevating direction may decrease from top to bottom.

The driving member 120 may be disposed adjacent to a lower portion of the elevating member 110 and may generate a driving force to move the needle N and the cannula C.

In example embodiments, the driving member 120 may include a spring having clockwise or counterclockwise rotational force using a longitudinal direction as a rotation axis.

The holder 130 may support the driving member 120. In example embodiments, the holder 130 may be coupled to the driving member 120, and thus rotate together in a clockwise or counterclockwise direction in clockwise or counterclockwise rotation of the driving member 120.

The holder 130 may include a holder body 131 including a lower portion supporting the driving member 120 and an upper portion protruding in the direction of the rotation axis of the driving member 120, a holder projection 132 formed on a bottom surface of the holder body 131 to protrude downwardly, a coupling groove (not shown) formed on the lower portion of the holder body 131, and a fixing groove (not shown) formed on the upper portion of the holder body 131. In example embodiments, the holder projection 132 may be provided eccentrically with respect to the rotation axis.

The driving member 120 may have a shape that surrounds the upper portion of the holder body 131 while being supported by the lower portion of the holder body 131.

The driving member 120 and the holder 130 may be coupled to each other as a bottom end of the driving member 120 is coupled to the coupling groove, such that the holder 130 may receive the rotational force of the driving member 120.

The holder 130 may be fixed as the fixing member 140 is positioned in the fixing groove, and may rotate as the fixing member 140 leaves the fixing groove.

The fixing member 140 may include a contact portion 141 that overlaps the elevating member 110 in the elevating direction, and a fixing portion 142 that overlaps the first sliding member 172 and the second sliding member 174 in the elevating direction.

In example embodiments, the contact portion 141 may contact the elevating member 110 when the elevating member 110 is elevated. In an embodiment, a surface of the contact portion 141 adjacent to the elevating member 110 may be inclined with respect to the elevating direction. In an embodiment, the width of the contact portion 141 in the horizontal direction may increase from top to bottom.

In example embodiments, the fixing portion 142 may be arranged to pass through the fixing hole 161 of the sliding rail 160. The fixing portion 142 may protrude from the fixing hole 161 to contact a surface of the second sliding member 174, and may fix the second sliding member 174.

In example embodiments, when the elevating member 110 is lowered, the contact portion 141 may contact the elevating projection 112 to move in the horizontal direction and in a first direction away from the sliding rail 160 by a lowering force of the elevating member 110. At this time, the fixing member 140 may leave the fixing groove such that the fixation of the holder 130 may be released. The fixing portion 142 may be released from contact with the second sliding member 174, thereby releasing the fixation of the second sliding member 174.

Details of how the fixing member 140 releases the fixation of the holder 130 and the fixation of the second sliding member 174 will be described in detail later.

The catching member 150 may include a body portion 151 coupled to one side of the sliding rail 160 and a catching portion 152 disposed on a front portion of the sliding rail 160.

The catching portion 152 may be disposed on a movement path of the first sliding member 172 and the second sliding member 174, and may include an inclined end and a horizontal end of the catching portion 152.

The sliding rail 160 may be disposed adjacent to the driving member 120 and the holder 130 and may provide the movement path for the first sliding member 172 and the second sliding member 174.

The first sliding member 172 and the second sliding member 174 may move linearly in one direction and the other direction on the sliding rail 160. At this time, the second sliding member 174 may be fixed at the extended position by being caught by the catching portion 152 disposed on the front portion of the sliding rail 160, and only the first sliding member 172 may be returned to its original position. Herein, one direction may refer to the direction of moving from the original position to the extended position, and the other direction may refer to the direction of moving from the extended position to the original position. Specific details regarding the movement of the first sliding member 172 and the second sliding member 174 will be described in detail later.

The first sliding member 172 may include a curved first slot hole 173 having a surface adjacent to the holder 130, the surface into which the holder projection 132 is inserted. In an embodiment, the first slot hole 173 may be curved in the one direction.

Meanwhile, the sliding rail 160 may include a curved second slot hole 162 having a surface adjacent to the holder 130, the surface into which the holder projection 132 is inserted. In an embodiment, the second slot hole 162 may be curved in the other direction.

In an embodiment, the first slot hole 173 and the second slot hole 162 may have substantially the same width.

Specific details regarding the movement of the holder projection 132 in the first slot hole 173 and the second slot hole 162 will be described in detail later.

The second sliding member 174 may include a catching projection 175 on one side, and the catching projection 175 may be fixed to the catching portion 152. The catching projection 175 may include an inclined surface and a vertical surface. Specific details regarding the fixation of the second sliding member 174 will be described in detail later.

The needle N may be fixed to the first sliding member 172, and the cannula C may be fixed to the second sliding member 174.

That is, the needle N may move together with the movement of the first sliding member 172, and the cannula C may move together with the movement of the second sliding member 174.

As the needle N is fixed to the first sliding member 172, the needle N may be inserted into or uninserted from the cannula C by the movement of the first sliding member 172. One end of the needle N may be connected to the reservoir unit 200 to deliver the medicine, and the other end thereof may be inserted into the cannula C to allow the medicine to move along the cannula C.

As the cannula C is fixed to the second sliding member 174, it may be inserted into the user's skin by the movement of the second sliding member 174. The cannula C has a conduit shape capable of accommodating the needle N, such that the medicine ejected from the needle N may be injected into the user.

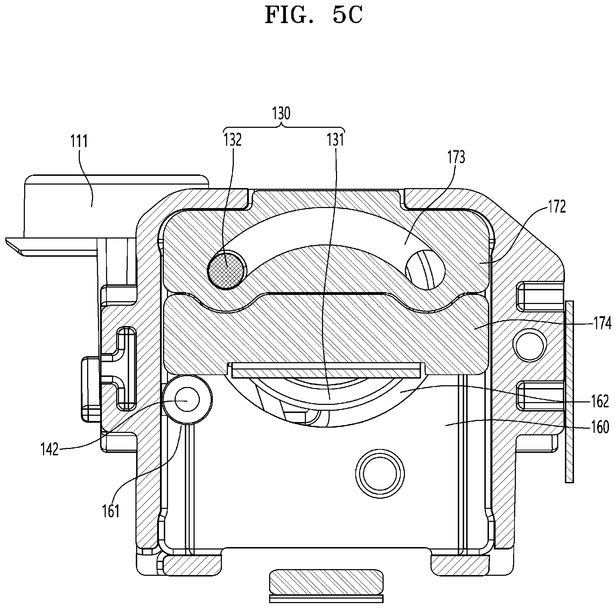

FIGS. 5A to 7C are side views and cross-sectional views showing a driving operation of the needle assembly. Specifically, FIGS. 5A, 5B, 6A, 6B, 7A, and 7B are side views showing the driving operation of the needle assembly, FIG. 5C is a cross-sectional view showing the driving operation of the needle assembly cut along line I-I of FIG. 4, and FIGS. 6C and 7C are cross-sectional views showing the driving operation of the needle assembly.

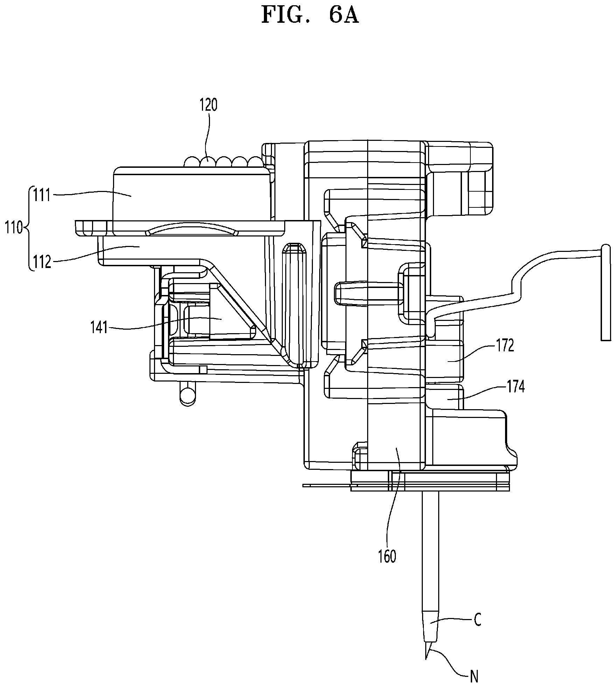

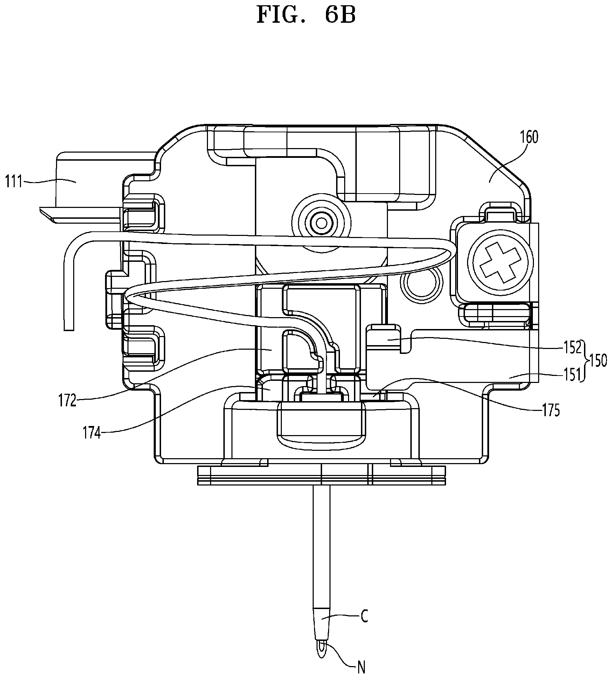

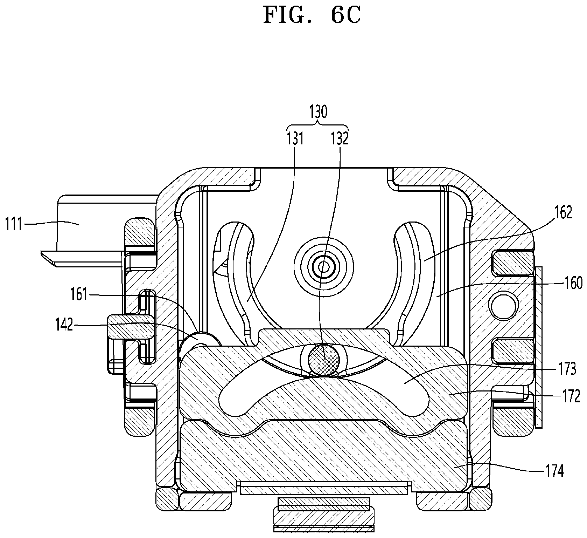

Meanwhile, for convenience of understanding the driving operation of the needle assembly, the button 101 is not shown in FIGS. 6A to 7C.

Referring to FIGS. 5A to 5C, the needle assembly 100 may be placed at its original position, that is, at a position before the user uses the needle assembly 100.

The fixing member 140 may be located in the fixing groove, and thus the holder 130 may be fixed by the fixing member 140. That is, before the user uses the needle assembly 100, the driving member 120 may be wound in the clockwise direction to have counterclockwise rotational force, but this rotational force may be suppressed by the holder 130 being fixed by the fixing member 140. That is, as the holder 130 is fixed, the first sliding member 172 connected to the holder 130 may also be fixed at its original position, and the second sliding member 174 located in the front portion of the first sliding member 172 may also be fixed at its original position.

At this time, the holder projection 132 may be located at one end of the first slot hole 172 and one end of the second slot hole 162.

Referring to FIGS. 6A to 6C, when the user lowers the elevating member 110 by pressing the button 101, the contact portion 141 of the fixing member 140 may contact the elevating projection 112 and move in the first direction by the lowering force of the elevating member 110, and in this case, the fixing member 140 may leave the fixing groove. Specifically, as a surface of the elevating projection 112 contacting the fixing member 140 is inclined with respect to the elevating direction, the width of a portion of the elevating projection 112 adjacent to the fixing member 140 in the horizontal direction decreases from top to bottom, a surface of the contact portion 141 contacting the elevating member 110 is inclined with respect to the elevating direction, and the width of the contact portion 141 in the horizontal direction increases from top to bottom, the contact portion 141 of the fixing member 140 may move in the horizontal direction by the lowering force of the elevating member 110.

In addition, the fixing portion 142 may be inserted into the fixing hole 161 as the contact portion 141 moves in the horizontal direction, such that the fixation of the second sliding member 174 by the fixing portion 142 may be released.

Accordingly, the holder 130 may rotate in the counterclockwise direction by the counterclockwise rotational force of the driving member 120. At this time, the holder projection 132 of the holder 130 may contact the first sliding member 172, such that the first sliding member 172 and the second sliding member 174 may move to the extended position in the counterclockwise rotation of the holder 130. At this time, the needle N fixed to the first sliding member 172 and the cannula C fixed to the second sliding member 172 may be inserted into the user's skin together.

Meanwhile, when the first sliding member 172 and the second sliding member 174 are located at the extended position, the rotational force of the driving member 120 may be about 50% of that when the driving member 120 is wound and fixed.

At this time, the holder projection 132 may be located in a center portion of the first slot hole 172 and a center portion of the second slot hole 162.

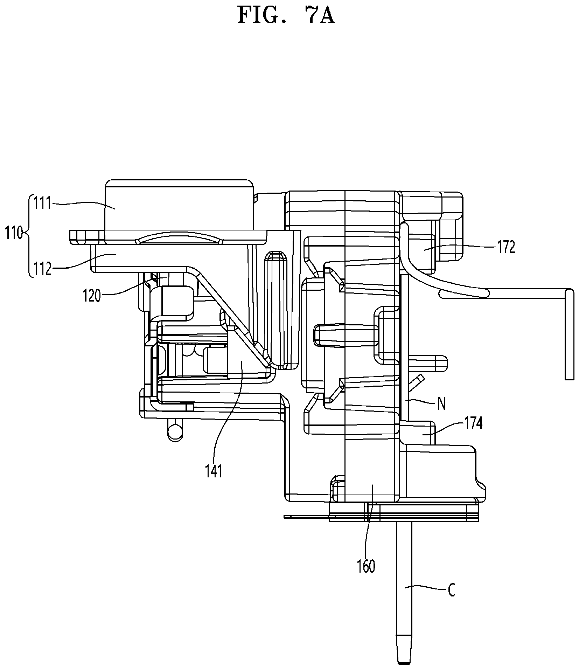

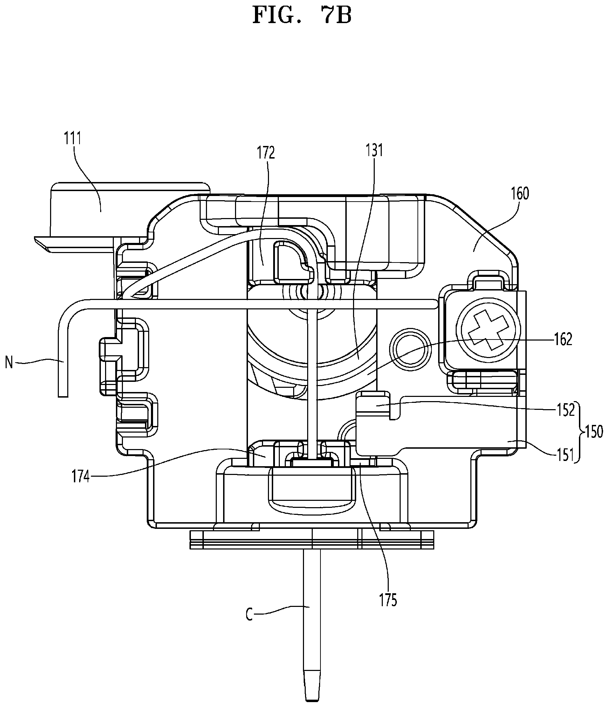

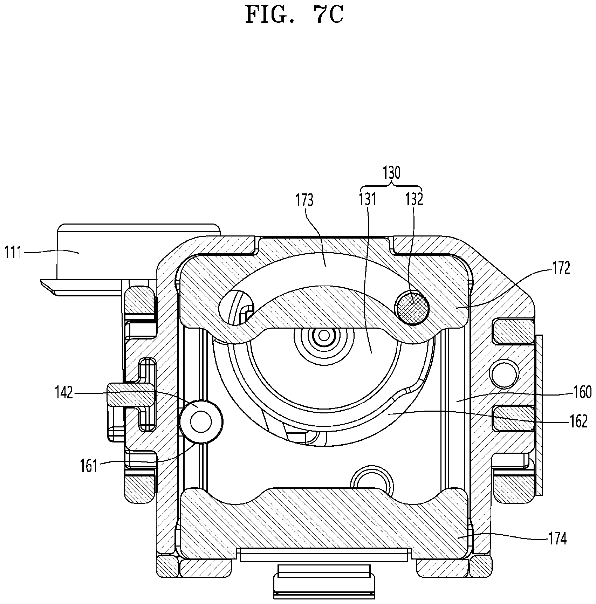

Referring to FIGS. 7A to 7C, the holder 130 may further rotate in the counterclockwise direction by the counterclockwise rotational force of the driving member 120. At this time, the first sliding member 172 contacting the holder projection 132 may return to its original position by the counterclockwise rotation of the holder 130. However, the second sliding member 174 may be fixed at the extended position by being caught by the catching portion 152 of the catching member 150. In example embodiments, the catching portion 152 may have a restoring force. In detail, while the inclined surface of the catching projection 175 passes under the catching portion 152, the catching portion 152 may be bent upward, but may soon return to its original shape due to the restoring force. Accordingly, the vertical surface of the catching projection 175 may be caught by the horizontal end of the catching portion 152, such that the second sliding member 174 may be fixed at the extended position by the catching portion 152.

Accordingly, the needle N fixed to the first sliding member 172 may be withdrawn from the user's skin, but the cannula C fixed to the second sliding member 174 may remain inserted into the user's skin. However, the needle N and the cannula C may be fluidly connected, such that the medical liquid injected from the reservoir unit 200 may be injected into the user through the needle N and the cannula C.

In an embodiment, when the first sliding member 172 returns to its original position, the contact portion 141 may move in the horizontal direction and in a second direction close to the sliding rail 160, and the elevating projection 112 may be elevated, such that the elevating member 110 may be elevated. Accordingly, the fixing member 140 may be positioned again in the fixing groove. Accordingly, as the holder 130 is fixed again by the fixing member 140, the first sliding member 172 may be fixed in its original position. Thus, it is possible to prevent the needle N from being inserted into the user's skin again.

In another embodiment, as the contact portion 141 does not move sufficiently in the second direction and the elevating projection 112 is not sufficiently elevated, the fixing member 140 may not be properly positioned back in the fixing groove. However, as the driving member 120 has exhausted all of its rotational force, it may not further rotate in the counterclockwise direction, and thus the first sliding member 172 may be fixed in its original position.

At this time, the holder projection 132 may be located at one end of the first slot hole 172 and the other end of the second slot hole 162.

Meanwhile, the insertion of the needle N and the cannula C into the user's skin may be performed as the user presses the button 101, but the withdrawal of the needle N from the user's skin may be automatically performed by the movement of the elevating member 110 and the fixing member 140, such that the operation of the needle assembly 100 may be performed by a user's single action of pressing the button 101. That is, as the operation of the needle assembly 100 may be performed efficiently, the probability of failure in the operation of the needle assembly 100 may be minimized.

Meanwhile, the operation of the needle assembly 100 may be performed by converting the rotational movement of the driving member 120 into the linear movement of the first sliding member 172 and the second sliding member 174.

In addition, since the holder projection 134 is provided eccentrically with respect to the rotation axis, the holder projection 131 may circularly move along the curved first slot hole 173 and second slot hole 162 during the movement of the first sliding member 172 and the second sliding member 174.

Additionally, the first slot hole 173 and the second slot hole 162 may overlap each other. In an embodiment, before and after the operation of the needle assembly 100, one end and the other end of the first slot hole 173 and one end and the other end of the second slot hole 162 may overlap, respectively. In an embodiment, during the operation of the needle assembly 100, the first slot hole 173 and the second slot hole 162 may overlap each other except for opposite ends thereof.

While it is illustrated in FIGS. 5A to 7C that the driving member 120 has the counterclockwise rotational force, and thus the description is made based on the illustration, the present disclosure is not necessarily limited thereto, and the driving member 120 may also have the clockwise rotational force.

Accordingly, the holder 130 may rotate in the clockwise direction by the clockwise rotational force of the driving member 120. In an embodiment, the first slot hole 173 may be curved in the other direction.

Referring back to FIGS. 2 and 3, the reservoir unit 200 may be mounted on a base body 50 and store medical liquid.

The reservoir unit 200 may have an inlet end and an outlet. The medical liquid may be injected into the inlet, the needle N may be installed in the outlet, and the medical liquid may be discharged through the needle N.

The driving module 300 may generate driving force and transmit the driving force to the driving unit 400.

When the driving units 400 are engaged with each other by the clutch unit 500, the driving module 300 may rotate the driving unit 400, and the medical liquid stored in the reservoir unit 200 may be discharged by the rotation of the driving unit 400.

The driving module 300 may use any type of device having a medical liquid suction force and a medical liquid ejection force by electricity. For example, any type of pump such as a mechanical displacement micropump, an electromagnetic motion micropump, etc., may be used. The mechanical displacement micropump may be a pump using motion of a solid or fluid such as a gear or diagram to cause a pressure difference for inducing flow of the fluid, and may include a diaphragm displacement pump, a fluid displacement pump, a rotary pump, etc. The electromagnetic motion micropump may be a pump using energy in an electric or magnetic form directly for movement of the fluid, and may include an electro hydrodynamic pump (EHD), an electro osmotic pump, a magneto hydrodynamic pump, an electro wetting pump, etc.

The trigger unit 600 may initiate the driving of the driving module 300 and connect the driving units 400 to each other. The trigger unit 600 may include first to third trigger members 610, 620, and 630, and may be rotatably disposed on the base body 50. When the user presses the button 101 of the needle assembly 100, the elevating member 110 may push the first trigger member 610 to start the rotation of the first trigger member 610, and the first trigger member 610 may rotate around the rotation axis to apply force to the clutch unit 500. At the same time, the first trigger member 610 may start the rotation of the second trigger member 620, and the second trigger member 620 may rotate about the rotation axis to apply force to the third trigger member 630. Thus, the third trigger member 630 may rotate about the rotation axis to apply force to the driving module 300. Accordingly, the trigger unit 600 may apply force to the driving module 300 to drive the driving module 300 and may apply force to the clutch unit 500 to connect the driving units 400 to each other.

The battery 700 may supply power to the liquid medicine injection apparatus 1. The battery 700 may be connected to the driving module 300 to control the driving of the driving module 300. The battery 700 may be rechargeable or disposable.

As described above, the operation of the needle assembly 100 may be efficiently performed by a user's single operation of pressing the button 101, thereby minimizing the probability of failure in the operation of the needle assembly 100.

Accordingly, the liquid medicine injection apparatus 1 including the needle assembly 100 may safely and simply inject a required amount of medical liquid into a user's body.

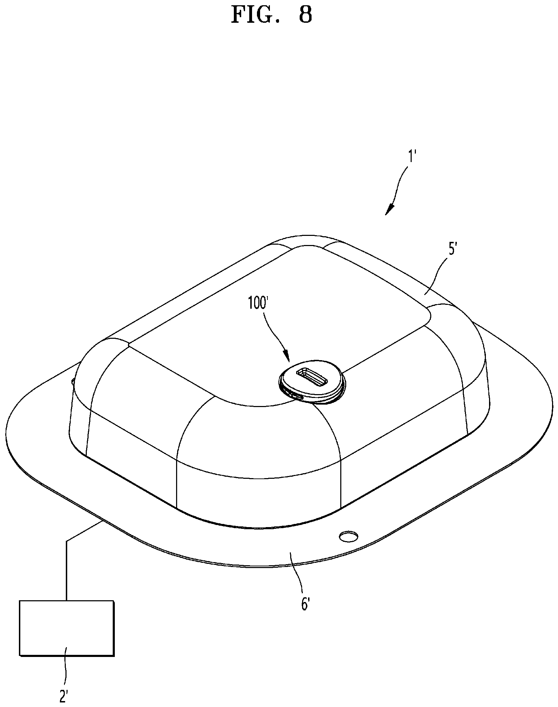

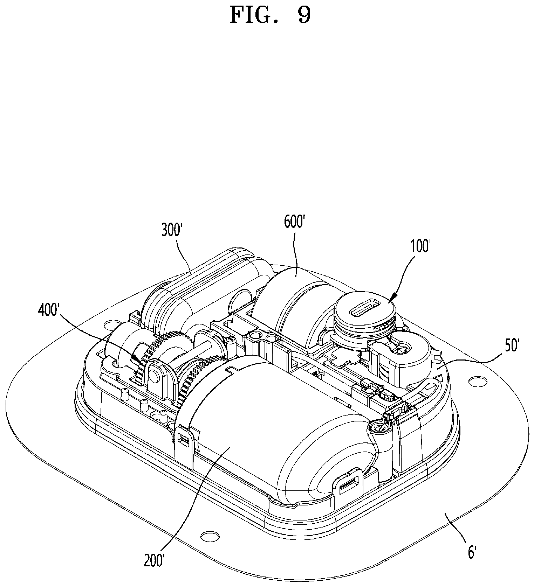

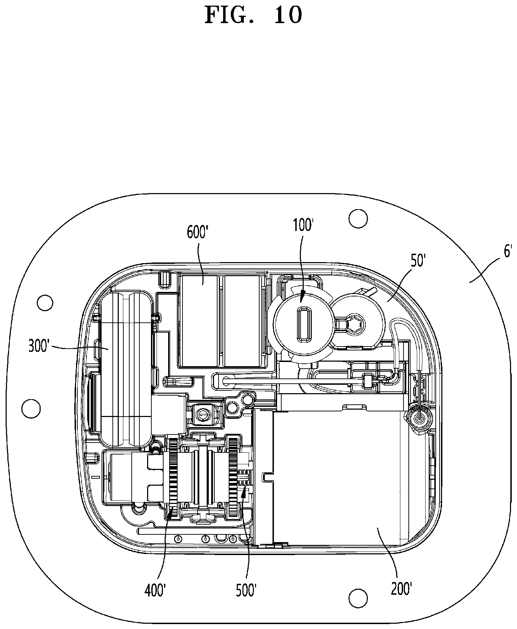

FIG. 8 is a perspective view showing a liquid medicine injection apparatus according to embodiments, FIG. 9 is a perspective view showing an internal arrangement of the liquid medicine injection apparatus of FIG. 8, and FIG. 10 is a plan view showing the liquid medicine injection apparatus of FIG. 9.

Referring to FIGS. 8 to 10, a liquid medicine injection apparatus 1' may be attached to the skin, etc., of the user and inject medical liquid stored therein into the user in a set required amount. The liquid medicine injection apparatus 1' may be used for various purposes according to a type of the medical liquid to be injected. The liquid medicine injection apparatus 1' may be mounted on not only humans but also animals to inject the medical liquid.

The liquid medicine injection apparatus 1' may be used for various purposes according to a type of the medical liquid to be injected. For example, the medical liquid may include insulin-based medical liquid for diabetic patients, and other various types of medical liquid such as medical liquid for pancreas, medical liquid for heart, etc.

The liquid medicine injection apparatus 1' may be connected to a remote device 2' wiredly or wirelessly connected thereto. The user may control the liquid medicine injection apparatus 1' and monitor a use state of the liquid medicine injection apparatus 1', by manipulating the remote device 2'. For example, the amount of the medical liquid injected from the liquid medicine injection apparatus 1', the number of injections of the medical liquid, the amount of the medical liquid stored in the reservoir unit 200, bio information of the user, etc., may be monitored, and based on the same, the user may drive the liquid medicine injection apparatus 1'.

In an embodiment, the remote device 2' may refer to a communication terminal that may use an application in a wired/wireless communication environment. Herein, the remote device 2' may be a portable terminal of the user. More specifically, the remote device 2' may include, but not limited to, a computer (e.g., a desktop, a laptop, a tablet, etc.), a media computing platform (e.g., a cable, a satellite set-top box, a digital video recorder), a handheld computing device (e.g., a PDA, an e-mail client, etc.), any type of a cellular phone, any type of a wearable device that may be used by being attached or mounted on a body of the user, or any other type of computing or communication platform.

The liquid medicine injection apparatus 1' and the remote device 2' may communicate with each other through a communication network. The communication network may refer to a communication network that provides a connection path such that the remote device 2' may transmit and receive data after connecting to a service server (not shown). The communication network may include wired networks such as local area networks (LANs), wide area networks (WANs), metropolitan area networks (MANs), integrated service digital networks (ISDNs), etc., or wireless networks such as wireless LANs, code division multiple access (CDMA), Bluetooth, satellite communication, etc., but the scope of the present disclosure is not limited thereto.

While it is shown in FIG. 1 that the remote device 2' is a single device, the present disclosure is not necessarily limited thereto and may include a plurality of devices capable of communicating with the liquid medicine injection apparatus 1'.

The liquid medicine injection apparatus 1' may include a housing 5' covering an outer side thereof and an attachment portion 6' positioned adjacent to the user's skin. The liquid medicine injection apparatus 1' may include a plurality of internal components arranged in an inner space between the housing 5' and the attachment portion 6'. A separate bonding means may be further provided between the attachment portion 6' and the user's skin, and the liquid medicine injection apparatus 1' may be fixed to the skin by the bonding means.

The liquid medicine injection apparatus 1' may include a base body 50', a needle assembly 100', a reservoir unit 200', a driving module 300', a driving unit 400', a clutch unit 500', and a battery 600'.

The base body 50' may form the basic frame of the housing 5', may be mounted in the internal space of the housing 5', and may fix the internal components of the liquid medicine injection apparatus 1' at a preset position. In an embodiment, the base body 50' may include a first body that covers a top side of the internal components and a second body that covers a bottom side of the internal components. In another embodiment, the base body 50' may be formed as one integral frame.

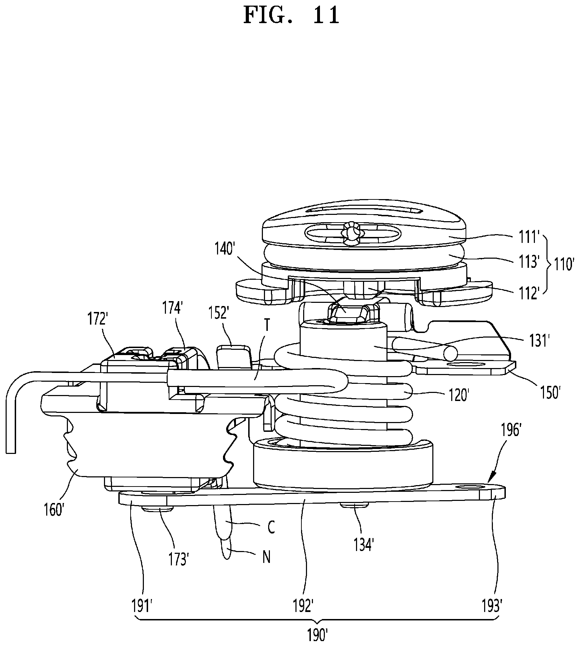

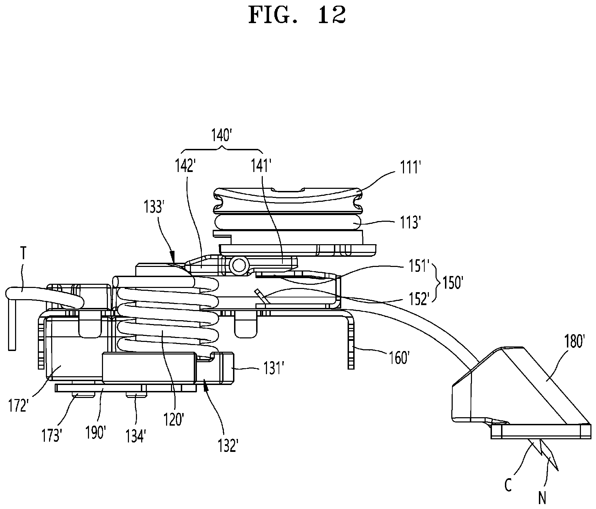

FIGS. 11 and 12 are side views showing the needle assembly of FIG. 9.

Referring to FIGS. 11 and 12, the needle assembly 100' may include the needle N, the cannula C, a driving member 120', a holder 130', and a connector 190'. Furthermore, the needle assembly 100' may further include a sleeve 110', a fixing member 140', a catching member 150', a sliding rail 160', a first sliding member 172', a second sliding member 174', a guide member 180', and the tube T.

The sleeve 110' may form the exterior of the needle assembly 100' and may be elevated. The sleeve 110' may include a elevating body 111', a elevating projection 112', and a sealing ring 113'.

The elevating body 111' may be elevated by user manipulation. In example embodiments, the elevating body 111' may include a groove in a top surface thereof. The user may manipulate the groove of the elevating body 111' exposed to outside to elevate the elevating body 111'.

The elevating projection 112' may be disposed adjacent to the fixing member 150' under the elevating body 111'. The elevating projection 112' may be elevated and lowered together as the elevating body 111' is elevated and lowered, such that the fixing member 150' may fix and rotate the holder 130'. Details of how the fixing member 150' fixes and rotates the holder 130' will be described in detail later.

The sealing ring 113' may be arranged to surround a part of an outer wall of the elevating body 111'.

In example embodiments, a space may be formed between the sleeve 110' and the housing 5' and the sealing ring 114' may fill the space. Accordingly, the sealing ring 114' may block external substances from being introduced into the liquid medicine injection apparatus 1' through the space, thereby preventing the internal components of the liquid medicine injection apparatus 1' from being contaminated.

In example embodiments, the sealing ring 113' may include a rubber material, and thus may have frictional force with the housing 5' when the sleeve 110' is elevated.

The driving member 120' may be disposed adjacent to a lower portion of the sleeve 110' and may generate a driving force to move the needle N and the cannula C.

In example embodiments, the driving member 120' may include a spring having clockwise or counterclockwise rotational force using a longitudinal direction as a rotation axis.

The holder 130' may support the driving member 120'. In example embodiments, the holder 130' may be coupled to the driving member 120', and thus rotate together in the clockwise or counterclockwise direction in clockwise or counterclockwise rotation of the driving member 120'.

The holder 130' may include a holder body 131' including a lower portion supporting the driving member 120' and an upper portion protruding in the direction of the rotation axis of the driving member 120', a coupling groove 132' formed on the lower portion of the holder body 131', a fixing groove 133' formed on the upper portion of the holder body 131', and a holder projection 134' formed on a bottom surface of the holder body 131' to protrude downwardly. In example embodiments, the holder projection 134' may be provided eccentrically with respect to the rotation axis.

The driving member 120' may have a shape that surrounds the upper portion of the holder body 131' while being supported by the lower portion of the holder body 131'.

The driving member 120' and the holder 130' may be coupled to each other as a bottom end of the driving member 120' is coupled to the coupling groove 132', such that the holder 130' may receive the rotational force of the driving member 120'.

The holder 130' may be fixed as the fixing member 140' is positioned in the fixing groove 133', and may rotate as the fixing member 140' leaves the fixing groove 133'.

The fixing member 140' may include a contact end 141' that is one end overlapping the sleeve 110' in the elevating direction in which the sleeve 110' is elevated, and a fixing end 142' that is the other end with respect to the contact end 141' and is positioned in and leaves the fixing groove 133'. In example embodiments, the contact end 141' may overlap the elevating projection 112' in the elevating direction. That is, when the sleeve 110' is lowered, the contact end 141' may contact the elevating projection 112' to be lowered together by the lowering force of the sleeve 110', and at this time, the fixing end 142' may rise and leave the fixing groove 133'.

The catching member 150', the sliding rail 160', and the first and second sliding members 170 and 180' may be disposed under the sleeve 110'.

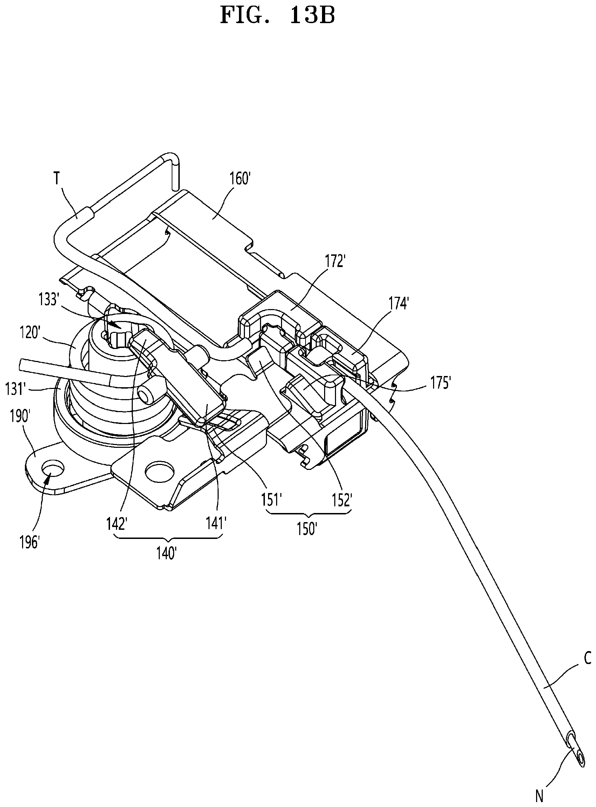

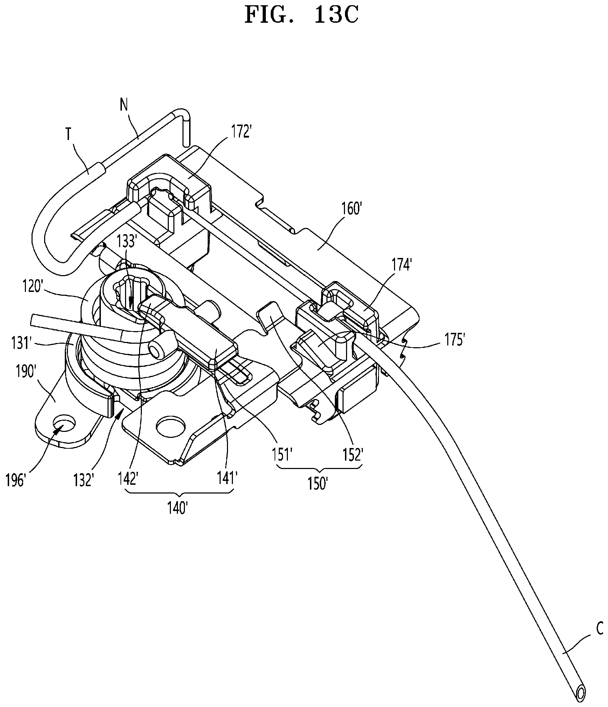

The catching member 150' may include a support portion 151' supporting the contact end 141' of the fixing member 140' and a catching portion 152' coupled to a front portion of the sliding rail 160'. The support portion 151' and the catching portion 152' may have restoring force. Accordingly, when the sleeve 110' is lowered and thus the contact end 141' is lowered, the support portion 151' may be bent in a lowering direction by the lowering force of the sleeve 110', and then the support portion 151' may return to its original form by the restoring force, and at this time, the contact end 141' may be elevated, and the fixing end 142' may be lowered and thus may be positioned again in the fixing groove 133' (see FIGS. 13B and 13C).

The catching portion 152' may be disposed on a movement path of the first sliding member 172' and the second sliding member 174', and may include an inclined end and a horizontal end of the catching portion 152'.

The sliding rail 160' may be disposed adjacent to the driving member 120' and the holder 130' and may provide the movement path for the first sliding member 172' and the second sliding member 174'. In example embodiments, the driving member 120' and the holder 130' may be spaced apart from the movement path of the first sliding member 172' and the second sliding member 174'. In an embodiment, the driving member 120' and the holder 130' may be arranged not to overlap the first sliding member 172' and the second sliding member 174' in the moving direction of the first sliding member 172' and the second sliding member 174'.

The first sliding member 172' and the second sliding member 174' may move linearly in one direction and the other direction on the sliding rail 160. At this time, the second sliding member 174' may be fixed at the extended position by being caught by the catching portion 152' disposed on the front portion of the sliding rail 160', and only the first sliding member 172' may be returned to its original position. Herein, one direction may refer to the direction of moving from the original position to the extended position, and the other direction may refer to the direction of moving from the extended position to the original position. Specific details regarding the movement of the first sliding member 172' and the second sliding member 174' will be described in detail later.

The first sliding member 172' may include a sliding projection 173' formed on the bottom surface thereof to protrude downwardly.

The second sliding member 174' may include a catching projection 175' on one side, and the catching projection' 175 may be fixed to the catching portion 152'. The catching projection 175' may include an inclined surface and a vertical surface. Specific details regarding the fixation of the second sliding member 174' will be described in detail later.

The needle N may be fixed to the first sliding member 172', and the cannula C may be fixed to the second sliding member 174'.

That is, the needle N may move together with the movement of the first sliding member 172', and the cannula C may move together with the movement of the second sliding member 174'.

As the needle N is fixed to the first sliding member 172', the needle N may be inserted into or uninserted from the cannula C by the movement of the first sliding member 172'. One end of the needle N may be connected to the reservoir unit 200' to deliver the medicine, and the other end thereof may be inserted into the cannula C to allow the medicine to move along the cannula C.

As the cannula C is fixed to the second sliding member 174', it may be inserted into the user's skin by the movement of the second sliding member 174'. The cannula C has a conduit shape capable of accommodating the needle N, such that the medicine ejected from the needle N may be injected into the user.

The needle N and cannula C may pass through the guide member 180' before being inserted into the user's skin. The guide member 180' may include a hole through which the needle N and the cannula C pass, and the hole may be inclined with respect to the user's skin.

Meanwhile, the first sliding member 172' and the second sliding member 174' move linearly on the sliding rail 160', such that the needle N fixed to the first sliding member 172' and the cannula C fixed to the second sliding member 174' move linearly on the sliding rail 160'. However, as the hole of the guide member 180' is inclined with respect to the user's skin, the needle N and the cannula C may be inclinedly inserted into the user's skin by the guide member 180'. Thus, when the needle N and the cannula C are inserted into the user's skin, damage to the user's skin may be minimized.

A portion between the reservoir unit 200' of the needle N and the first sliding member 172' may be covered by the tube T. The tube T may have a conduit shape capable of accommodating the needle N, and thus may move together with the needle N when the first sliding member 172' moves. Additionally, the tube T may prevent the needle N from being twisted or tangled with surrounding components in the operation of the needle assembly 100'.

The connector 190' may be disposed between the holder 130' and the needle N. The connector 190' may contact the holder 130' and the first sliding member 172' and connect them to each other.

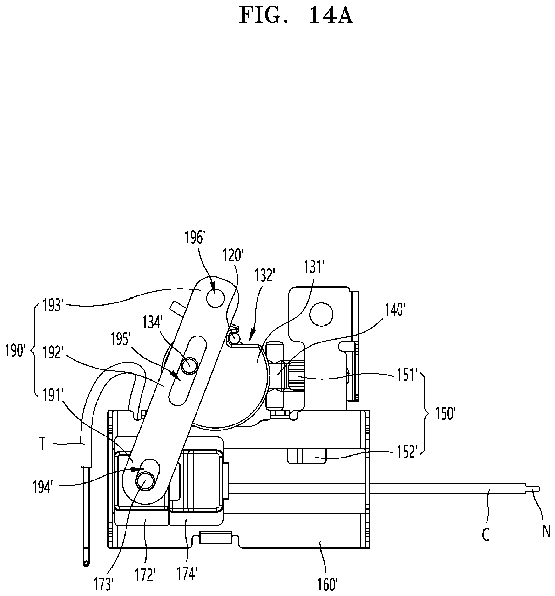

The connector 190' may include a first end 191' contacting the first sliding member 172', a central portion 192' contacting the holder 130', and a second end 193' that is a rotation axis. The first end 191', the central portion 192', and the second end 193' may respectively include a first slot hole 194', a second slot hole 195', and a regular hole 196', the first sliding projection 173' may be inserted into the first slot hole 194', and the holder projection 134' may be inserted into the second slot hole 195' (see FIG. 14A).

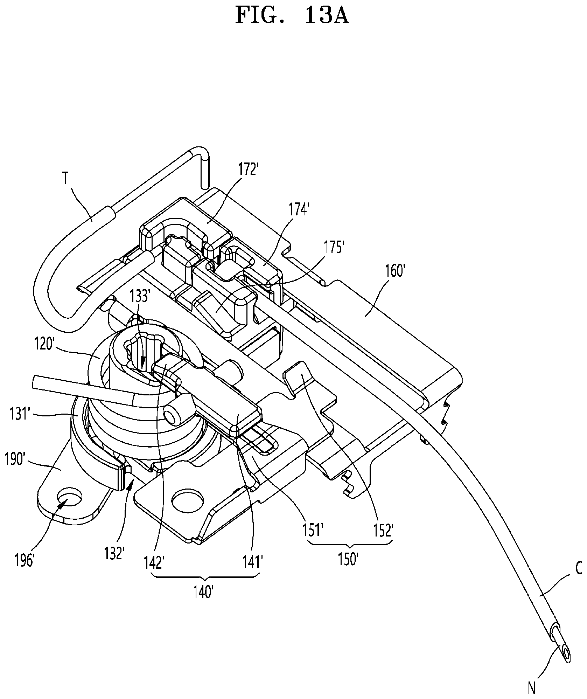

FIGS. 13A to 13C are perspective views showing a driving operation of the needle assembly, and FIGS. 14A to 14C are bottom views showing the driving operation of the needle assembly.

For convenience of understanding the operation, the sleeve 110' and the guide member 180' are removed and shown in FIGS. 13A to 14C.

Referring to FIGS. 13A and 14A, the needle assembly 100' may be placed at its original position, that is, at a position before the user uses the needle assembly 100'.

The fixing member 140' may be located in the fixing groove 133', and thus the holder 130' may be fixed by the fixing member 140'. That is, before the user uses the needle assembly 100', the driving member 120' may be wound in the counterclockwise direction to have the clockwise rotational force, but this rotational force may be suppressed by the holder 130' being fixed by the fixing member 140'. That is, as the holder 130' is fixed, the first sliding member 172' connected to the holder 130' by the connector 190' may also be fixed at its original position, and the second sliding member 174' located in the front portion of the first sliding member 172' may also be fixed at its original position.

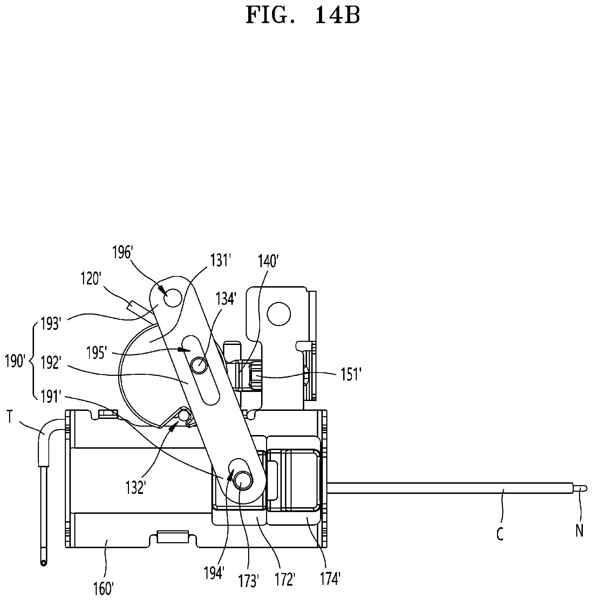

Referring to FIGS. 13B and 14B, when the user presses the sleeve 110' to lower the sleeve 110', the contact end 141' of the fixing member 140' may contact the elevating projection 112' and may be lowered together by the lowering force of the sleeve 110', and in this case, the fixed end 142' of the fixing member 140' may be elevated and leave the fixing groove 133'. At this time, the support portion 151' of the catching member 150' may be bent in the lowering direction due to the lowering of the contact end 141'.

Accordingly, the holder 130' may rotate in the clockwise direction by the clockwise rotational force of the driving member 120'. At this time, as a central portion 192' of the connector 190' contacts a holder projection 134' of the holder 130', the connector 190' may rotate in the clockwise direction by the clockwise rotation of the holder 130'. In addition, the sliding projection 173' of the first sliding member 172' contacts the first end 191' of the connector 190', such that the first sliding member 172' and the second sliding member 174' located in front of the first sliding member 172' may move to the extended position by the clockwise rotation of the connector 190'. At this time, the needle N fixed to the first sliding member 172' and the cannula C fixed to the second sliding member 172' may be inserted into the user's skin together.

Meanwhile, when the first sliding member 172' and the second sliding member 174' are located at the extended position, the rotational force of the driving member 120 may be about 50% of that when the driving member 120' is wound and fixed.

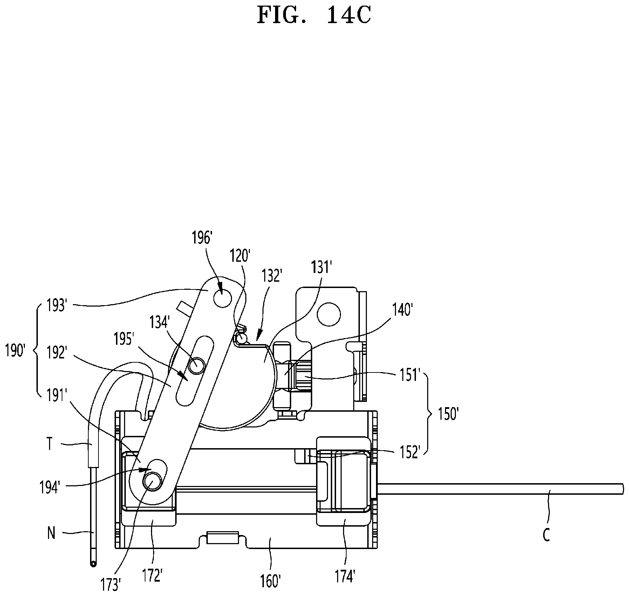

Referring to FIGS. 13C and 14C, when the holder 130' further rotates in the clockwise direction due to the clockwise rotational force of the driving member 120', the connector 190' may rotate in the counterclockwise direction by the clockwise rotation of the holder 130'. At this time, the first sliding member 172' contacting the connector 190' may return to its original position by the counterclockwise rotation of the connector 190'. However, the second sliding member 174' may be fixed at the extended position by being caught by the catching portion 152' of the catching member 150'. In detail, while the inclined surface of the catching projection 175' passes under the catching portion 152', the catching portion 152' may be bent upward, but may soon return to its original shape due to the restoring force. Accordingly, the vertical surface of the catching projection 175' may be caught by the horizontal end of the catching portion 152', such that the second sliding member 174' may be fixed at the extended position by the catching portion 152'.

Accordingly, the needle N fixed to the first sliding member 172' may be withdrawn from the user's skin, but the cannula C fixed to the second sliding member 174' may remain inserted into the user's skin. However, the needle N and the cannula C may be fluidly connected, such that the medical liquid injected from the reservoir unit 200' may be injected into the user through the needle N and the cannula C.

That is, the operation of the needle assembly 100' may be performed in such a manner that the connector 190' moves the needle N and the cannula C in a direction when the holder 130' rotates, and the connector 190' moves only the needle N in the other direction when the holder 130' further rotates.

In an embodiment, when the first sliding member 172' returns to its original position, the contact end 141' may be elevated by the restoring force of the support portion 151', and in this case, the fixed end 142' may be lowered and positioned again in the fixing groove 133'. Accordingly, as the holder 130' is fixed again by the fixing member 140', the first sliding member 172' may be fixed in its original position. Thus, it is possible to prevent the needle N from being inserted into the user's skin again. Meanwhile, when the contact end 141' is elevated, the sleeve 110' may be elevated together by the elevating force of the contact end 141'.

In another embodiment, when the frictional force between the sealing ring 113' of the sleeve 110' and the housing 5' is greater than the elevating force of the contact end 141', the elevation of the contact end 141' may be limited. That is, the fixed end 142' may not be lowered sufficiently and thus may not be properly positioned back in the fixed groove 133'. However, as the driving member 120' has exhausted all of its rotational force, it may not further rotate in the clockwise direction, and thus the first sliding member 172' may be fixed in its original position.

Meanwhile, the insertion of the needle N and the cannula C into the user's skin may be performed as the user presses the sleeve 110', but the withdrawal of the needle N from the user's skin may be automatically performed by the restoring force of the support portion 151', such that the operation of the needle assembly 100' may be performed by a user's single action of pressing the sleeve 110'. That is, as the operation of the needle assembly 100' may be performed efficiently, the probability of failure in the operation of the needle assembly 100' may be minimized.

Meanwhile, the operation of the needle assembly 100' may be performed by converting the rotational movement of the driving member 120' into the linear movement of the first sliding member 172' and the second sliding member 174'. In example embodiments, a position when the first sliding member 172' of the connector 190' returns to its original position may be the same as a position before the first sliding member 172' and the second sliding member 174' move.

In addition, as the holder projection 134' is provided eccentrically with respect to the rotation axis, during the movement of the first sliding member 172' and the second sliding member 174', the sliding projection 173' may linearly move along the first slot hole 194' and the holder projection 134' may linearly move along the second slot hole 195'.

Moreover, as the rotational force of the driving member 120' is gradually exhausted as the driving member 120' rotates, the movement speed of the first sliding member 172' and the second sliding member 174' to the extended position may be different from the movement speed of the first sliding member 172' returning to its original position. That is, the movement speed of the first sliding member 172' and the second sliding member 174' to the extended position may be higher than the movement speed of the first sliding member 172' returning to its original position. In addition, the insertion speed of the needle N and the cannula C into the user's skin may be higher than the withdrawal speed of the needle N from the user's skin, thereby minimizing damage to the user's skin.

In example embodiments, the shape of the connector 190' may not change during the movement of the first sliding member 172' and the second sliding member 174', and the second end 193', which is the rotation axis, may be fixed.

Meanwhile, while a description has been made based on the illustration of FIGS. 13A to 14C that the driving member 120' has the clockwise rotational force and is disposed to the right of the first sliding member 172' and the second sliding member 174' with respect to the movement direction of the first sliding member 172' and the second sliding member 174', the present disclosure is not limited to the description, such that the driving member 120' may have the counterclockwise rotational force and may be disposed to the left of the first sliding member 172' and the second sliding member 174' with respect to the movement direction of the first sliding member 172' and the second sliding member 174'.

Accordingly, the holder 130' may rotate in the counterclockwise direction by the counterclockwise rotational force of the driving member 120'. At this time, as a central portion 192' of the connector 190' contacts a holder projection 134' of the holder 130', the connector 190' may rotate together in the counterclockwise direction by the counterclockwise rotation of the holder 130'. In addition, the sliding projection 173' of the first sliding member 172' contacts the first end 191' of the connector 190', such that the first sliding member 172' and the second sliding member 174' located in front of the first sliding member 172' may move to the extended position by the counterclockwise rotation of the connector 190'.

Thereafter, when the holder 130' further rotates in the counterclockwise direction due to the counterclockwise rotational force of the driving member 120', the connector 190' may rotate in the clockwise direction by the counterclockwise rotation of the holder 130'. At this time, the first sliding member 172' may return to its original position by the clockwise rotation of the connector 190'.

Referring back to FIGS. 9 and 10, the reservoir unit 200' may be mounted on the base body 50' and store medical liquid.

The reservoir unit 200' may have an inlet end and an outlet. The medical liquid may be injected into the inlet, the needle N may be installed in the outlet, and the medical liquid may be discharged through the needle N.

The driving module 300' may generate driving force and transmit the driving force to the driving unit 400'.

When the driving units 400' are engaged with each other by the clutch unit 500', the driving module 300' may rotate the driving unit 400', and the medical liquid stored in the reservoir unit 200' may be discharged by the rotation of the driving unit 400'.

The driving module 300' may use any type of device having a medical liquid suction force and a medical liquid ejection force by electricity. For example, any type of pump such as a mechanical displacement micropump, an electromagnetic motion micropump, etc., may be used. The mechanical displacement micropump may be a pump using motion of a solid or fluid such as a gear or diagram to cause a pressure difference for inducing flow of the fluid, and may include a diaphragm displacement pump, a fluid displacement pump, a rotary pump, etc. The electromagnetic motion micropump may be a pump using energy in an electric or magnetic form directly for movement of the fluid, and may include an electro hydrodynamic pump (EHD), an electro osmotic pump, a magneto hydrodynamic pump, an electro wetting pump, etc.

In example embodiments, the driving module 300' may be electrically connected to the remote device 2', and the driving module 300' may operate through control of the remote device 2'.

The battery 600' may supply power to the liquid medicine injection apparatus 1'. The battery 600' may be connected to the driving module 300' to control the driving of the driving module 300'. The battery 600' may be rechargeable or disposable.

As described above, as the hole of the guide member 180' is inclined with respect to the user's skin, the needle N and the cannula C may be inclinedly inserted into the user's skin by the guide member 180'. In addition, the insertion speed of the needle N and the cannula C into the user's skin may be higher than the withdrawal speed of the needle N from the user's skin. Thus, when the needle N and the cannula C are inserted into the user's skin, damage to the user's skin may be minimized when the needle N is withdrawn from the user's skin.

The operation of the needle assembly 100' may also be efficiently performed by a user's single operation of pressing the sleeve 110', thereby minimizing the probability of failure in the operation of the needle assembly 100'.

Accordingly, the liquid medicine injection apparatus 1' including the needle assembly 100' may safely and simply inject a required amount of medical liquid into a user's body.

Although the present disclosure has been described above with reference to embodiments, it may be understood by those of ordinary skill in the art that various modifications and changes may be made to the present disclosure without departing from the spirit and scope of the present disclosure as set forth in the claims below.

### Industrial Applicability

The present disclosure relates to a needle assembly and a liquid medicine injection apparatus comprising same. Embodiments of the present disclosure may be applied to a needle assembly and a liquid medicine injection apparatus for injecting a medical liquid used in industry.

## Claims

1. A needle assembly comprising:
a needle;
a cannula into which the needle is inserted;
a driving member generating driving force to move the needle and the cannula; and
a holder on which the driving member is supported,
wherein the holder rotates to move the needle and the cannula in a direction, and the holder further rotates to move the needle in another direction.

2. The needle assembly of claim 1, further comprising:
a first sliding member to which the needle is fixed; and
a second sliding member to which the cannula is fixed and which is disposed adjacent to the first sliding member,
wherein the holder is connected to the first sliding member.

3. The needle assembly of claim 2, further comprising
a catching member disposed on a movement path of the first sliding member and the second sliding member, wherein the second sliding member is caught and fixed to the catching member.

4. The needle assembly of claim 2, wherein the holder comprises, on a
bottom surface thereof, a holder projection protruding downwardly, and the first sliding member comprises, on a surface thereof, a curved first slot hole into which the holder projection is inserted.

5. The needle assembly of claim 4, further comprising a sliding rail providing
a movement path for the first sliding member and the second sliding member,
wherein the sliding rail comprises, on a surface thereof, a curved second slot hole into which the holder projection is inserted.

6. The needle assembly of claim 5, wherein the holder projection moves
circularly along the first slot hole and the second slot hole.

7. A liquid medicine injection apparatus comprising:
a base body;
a reservoir unit mounted on the base body and storing medical liquid;
a needle assembly mounted on the base body and injecting medical liquid into a body of a user; and
a driving module connected to the reservoir unit to transfer the medical liquid of the reservoir unit to the needle assembly,
wherein the needle assembly comprises:
a needle;
a cannula into which the needle is inserted;
a driving member generating driving force to move the needle and the cannula; and
a holder on which the driving member is supported,
wherein the holder rotates to move the needle and the cannula in a direction, and the holder further rotates to move the needle in another direction.
